(19) Europäisches Patentamt
European Patent Office
Office européen des brevets

(11) **EP 3 027 177 B1**

(12) **EUROPEAN PATENT SPECIFICATION**

(45) Date of publication and mention
of the grant of the patent:
**12.07.2017 Bulletin 2017/28**

(51) Int Cl.:
***A61K 9/20*** (2006.01)  ***A61K 31/64*** (2006.01)
***A61P 3/10*** (2006.01)

(21) Application number: **14757858.7**

(86) International application number:
**PCT/EP2014/066599**

(22) Date of filing: **01.08.2014**

(87) International publication number:
**WO 2015/014987 (05.02.2015 Gazette 2015/05)**

(54) **A MODIFIED-RELEASE ORAL PHARMACEUTICAL FORMULATION CONTAINING GLICLAZIDE**

ORALE PHARMAZEUTISCHE GLICLAZIDHALTIGE FORMULIERUNG MIT MODIFIZIERTER FREISETZUNG

FORMULATION PHARMACEUTIQUE ORALE À LIBÉRATION MODIFIÉE CONTENANT DU GLICLAZIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2013 IT FI20130184**

(43) Date of publication of application:
**08.06.2016 Bulletin 2016/23**

(73) Proprietor: **VALPHARMA INTERNATIONAL S.P.A.**
**47864 Pennabilli (IT)**

(72) Inventors:
• **VALDUCCI, Roberto**
  **I-47039 Savignano Sul Rubicone (IT)**
• **AVANESSIAN, Serozh**
  **I-47827 Villa Verucchio (IT)**

(74) Representative: **Valenza, Silvia et al**
**Notarbartolo & Gervasi S.p.A.**
**Corso di Porta Vittoria 9**
**20122 Milano (IT)**

(56) References cited:
WO-A1-00/18373          WO-A1-2006/061697
WO-A1-2006/123213       WO-A1-2009/098195
WO-A2-03/026637         WO-A2-2008/062470
US-A1- 2009 017 116     US-A1- 2009 238 870

• **LABANA M ET AL: "Formulation and in vitro evaluation of modified release Gliclazide tablet", JOURNAL OF CHEMICAL AND PHARMACEUTICAL RESEARCH 2011 JOURNAL OF CHEMICAL AND PHARMACEUTICAL RESEARCH IND , vol. 3, no. 3 2011, pages 348-352, XP002718236, ISSN: 0975-7384 Retrieved from the Internet: URL:http://jocpr.com/vol3-iss3-2011/JCPR-2 011-3-3-348-352.pdf [retrieved on 2013-12-20]**
• **FERDOUS R ET AL: "In vitro release kinetic study of gliclazide from methocel K100 MCR and methocel k100 LVCR matrix tablets", INTERNATIONAL JOURNAL OF PHARMTECH RESEARCH 2012 SPHINX KNOWLEDGE HOUSE IND , vol. 4, no. 2 April 2012 (2012-04), pages 883-888, XP002718237, ISSN: 0974-4304 Retrieved from the Internet: URL:http://www.researchgate.net/publicatio n/225285599_In_vitro_Release_Kinetic_Study _of_Gliclazide_from_Methocel_K_100_MCR_an d _Methocel_K100_LVCR_Matrix_Tablets/file/9f cfd4fd62a24a03bb.pdf [retrieved on 2013-12-20]**

## Description

Field of the Invention

The present invention relates to a pharmaceutical composition comprising Gliclazide, in particular sustained-release tablets containing Gliclazide.

State of the Art

Gliclazide is a second generation hypoglycemic sulfonylurea used in the treatment of diabetes. It is usually administered by oral route in controlled-release formulations capable of making the active ingredient available for absorption.

In WO 00/18373 A1 a controlled-release Gliclazide tablet is described, said tablet comprising 12-40% w/w of Gliclazide, 10-40% w/w of a hydroxypropylmethyl cellulose (HPMC) 4000cps and HPMC 100cps mixture, 2-20% w/w of maltodextrin and 35-75% w/w of $CaHPO_4$ as diluent, wherein % w/w referred to the total weight of the tablet.

The patent WO 2006/123213 refers to a formulation obtained by granulation (dry or wet) of Gliclazide with addition of binders and other excipients. In order to modulate the release of Gliclazide, a mixture of two types of HPMC (HPMC K100LV and HPMC K4M CR) in extra-granular phase is added to this granulate. The patent WO 2006/061697 describes a formulation containing Gliclazide, with particle size lower than 50 microns, a mixture of two HPMC polymers (HPMC K100LV and HPMC K4M CR) to control the release, and a mono- and/or a disaccharide (lactose) added to the formulation by wet granulation.

The patent WO 2008/062470 describes a formulation containing Gliclazide granulated with HPMC K4M, $CaHPO_4$, and a solution of PVP K30 in iPrOH, and it does not include any saccharide component.

All the previous formulations have in common the use of calcium phosphate dibasic anhydrous or dihydrate as diluent.

All previous formulations show a substantially pH-independent release kinetics at pH between 4 and 8.

WO03/026637 describes (in Table 5 or 7) a tablet comprising metformin and a mixture of two cellulose derivatives (specifically HPMC K4M and HPMC K100M) in the core. A third cellulose derivative (specifically Methocel E5) is not in the core but in a layer coating the core.

US2009/238870 (equivalent of EP2103302) describes a gliclazide tablet containing two low viscosity HPMCs (see table in §[45]).

Labana et al. (J. Chem. Pharm. Res. 2011, 3(3),348-352) describes a gliclazide tablet containing a medium viscosity HPMC (400 cps) and a high viscosity HPMC (K100M).

Summary of the Invention

The object of the present invention is a Gliclazide sustained-release pharmaceutical composition; said composition consisting of:

Gliclazide, as active ingredient, mixed with;
a mixture of hydroxypropylmethyl cellulose (HPMC) said mixture comprising at least two different HPMCs having medium or high viscosity, and at least one HPMC having low viscosity;
other diluents, all soluble in water; and
optionally a glidant and/or a lubricant;
wherein medium viscosity means a viscosity not lower than 2000cps, high viscosity means a viscosity higher than 50000cps, and low viscosity means a viscosity lower than 100cps.

The composition object of the invention is devoid of $CaHPO_4$.

The formulation object of the present patent shows a pH-dependent release kinetics, and in particular it shows a release comparable to that of the leading product of reference (described in EP2103302, and commercially available as Diamicron®) at pH 6.8, while at pH 4.5 and in water, the release profile of the formulation deviates significantly from the results obtained with the official method of analysis at pH 6.8. One may therefore state that this formulation has, unlike the formulations known in the art, a pH dependence.

Detailed Description of the Invention

The pharmaceutical composition according to the invention is for Gliclazide oral administration. The cellulose derivative of the invention is hydroxypropylmethyl cellulose (HPMC). The mixture of cellulose derivatives, according to the invention, preferably has a total viscosity between 6000 and 21000 cps.

Preferably, the mixture of cellulose derivatives is consisting of three HPMC of which: one HPMC having viscosity higher than 50000cps, one HPMC having viscosity of between 2000 and 50000 cps, and one HPMC having viscosity lower than 100 cps.

More preferably, the mixture of cellulose derivatives is consisting of:

one HPMC having viscosity higher than 70000cps, one HPMC having viscosity of between 3000 and 30000 cps, and one HPMC having viscosity lower than 50 cps. More preferably, the three HPMC are selected from HPMC 5cps, HPMC 4000cps, HPMC 15000cps, and HPMC 100000cps.

The composition has an active ingredient content between 15% and 20% w/w with respect to the total of the composition, wherein the release is controlled by the polymer matrix consisting of the mixture of HPMC, mixed together in order to obtain a porous matrix and fast hydration. As preferred embodiment, this was obtained by mixing two high or medium viscosity HPMC with a low viscosity HPMC in percentages ranging between 20 and 40% w/w with respect to the total weight of the formulation, more preferably 30% w/w of the finished tablet. Preferably, the low viscosity HPMC is present in amounts ranging between 7% w/w and 15% w/w with respect to the total of the formulation. Surprisingly, it has been found that the presence of low viscosity HPMC associated with high viscosity HPMC leads to a higher initial hydration of the matrix resulting in a slower early hours release kinetics than the combination of only two HPMC with a viscosity higher than 4000cps.

Surprisingly, the addition of low viscosity HPMC to a mixture of two medium and/or high viscosity HPMC increases the hydration of the system carrying a synergistic action with other soluble excipients present in the formulation. This effect slows down the release in the first 4h, after which the release is controlled by the presence of the other two more viscous HPMC.

The absence of calcium phosphate dibasic does not affect the release.

The presence of water-soluble diluents, between 40 and 60% w/w with respect to the total weight of the composition (more preferably 50%), leads to a further increase of the canalization and, therefore, the hydration of the matrix. Preferably said water-soluble diluents are selected from the group consisting of polyalcohols and mixtures thereof; in particular mannitol, maltodextrin, sorbitol, isomalt, and mixtures thereof.

Surprisingly, at pH 6.8, a release identical to the one of the leading product has been obtained without any addition of calcium phosphate dibasic to the composition, and using excipients all soluble. In addition to the active ingredient, the polymers and the soluble diluents, in the formulation also a glidant and a lubricant may be included, in order to improve the workability. Said glidant is preferably chosen from the group consisting of magnesium stearate, sodium stearyl fumarate, stearic acid, or other suitable glidants, or mixture thereof. Said lubricant is preferably chosen from the group consisting of Anhydrous Colloidal Silica, or other suitable lubricants, or mixture thereof. If present, glidant and lubricant percentages are not exceeding 1% w/w with respect to the total of the composition.

In a preferred manner, the composition object of the present invention has the following % w/w composition with respect to the total of the composition:

| | |
|---|---|
| Gliclazide | 15-20%; |
| mixture of cellulose derivatives | 20-40%; |
| water-soluble diluents | 40-60%; |
| glidant | 0-1%; |
| lubricant | 0-1%. |

In a particularly preferred manner, the composition object of the present invention has the following % w/w composition with respect to the total of the composition:

| | |
|---|---|
| Gliclazide | 15-20%; |
| HPMC 5cps | 7-15%; |
| HPMC 100000cps | 6-12%; |
| HPMC 4000cps | 4-8%; |
| Mannitol | 20-30%; |
| Maltodextrin | 20-30%; |
| Magnesium Stearate | 0.30-0.60%; |
| Anhydrous Colloidal Silica | 0.50-1.00%. |

The composition subject-matter of the present invention is preferably in the form of tablets obtained by direct compression or by induction granulation. In a particularly preferred embodiment, the invention relates to 60mg Gliclazide tablets, said tablets being divisible into two or more dosing fractions. It has been observed that tablets divided into two half-doses show, in turn, a different release profile to the whole tablet but, surprisingly, comparable to the kinetics of other 30mg Gliclazide formulations known in the art (see EP2103302).

Preferably, the composition object of the present invention may be prepared by mixing Gliclazide to the mixture of cellulose derivatives in order to obtain a mixture to which the water-soluble diluents and finally the glidant and the lubricant are added. The tablets production method may be a direct compression of the mixture of all components described above. In particular, in a preferred embodiment of the invention, the tablets are oblong in shape and equipped with a fracture line to facilitate the divisibility in half.

BRIEF DESCRIPTION OF THE FIGURES

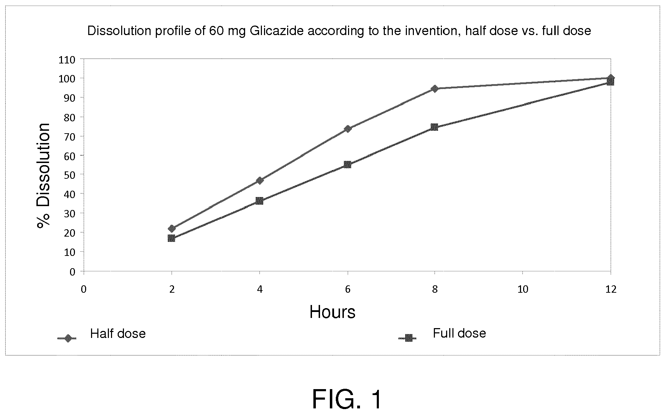

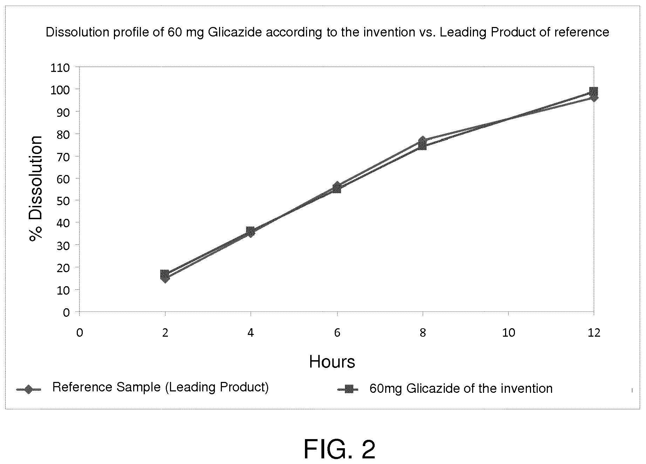

FIG. 1 shows the dissolution profile of the tablets according to the invention: full dose (60mg) and half dose (30mg). FIG.2 shows the dissolution profile of the tablets according to the invention in comparison to a reference tablets known in the art.

Example 1

Manufacturing of 60 mg/CPR Gliclazide divisible tablets

The percentages of the components used in the formulation are shown in the following table:

| Component | % |
| --- | --- |
| Gliclazide | 18.7 |
| HPMC 5cps | 13.5 |
| HPMC 100000cps | 10.5 |
| HPMC 4000cps | 6 |
| Mannitol | 25 |
| Maltodextrin | 25 |
| Magnesium Stearate | 0.45 |
| Anhydrous Colloidal Silica | 0.85 |

Gliclazide is added into a high speed granulator together with the three HPMC in order to incorporate in an optimal manner the active ingredient into the polymers that will form the matrix. To this mixture, always into the granulator, Mannitol and Maltodextrin, which will act as diluents and channelling agents, are added. Finally, sieved Anhydrous Colloidal Silica and Magnesium Stearate are added into the bin. The mixture obtained is then compressed by means of a rotary tablet press fitted with oblong punches with a fracture line.

The releases of the tablets obtained with this method are given below.

Comparison of dissolution profiles of half dose of 60mg Gliclazide according to the invention vs. full dose of 60mg Gliclazide according to the invention:

Dissolution medium: phosphate buffer pH 6.8, 1000 ml
Apparatus: stationary basket, 50 rpm

Reference sample: half dose of 60mg Gliclazide tablet

| Hours | R1 | R2 | R3 | R4 | R5 | R6 | R average | DSR |
| --- | --- | --- | --- | --- | --- | --- | --- | --- |
| 2 | 21.5 | 20.9 | 21.0 | 22.4 | 22.3 | 23.9 | 22.0 | 5.1 |
| 4 | 45.9 | 45.9 | 45.4 | 46.0 | 47.1 | 49.9 | 46.7 | 3.6 |

(continued)

| Hours | R1 | R2 | R3 | R4 | R5 | R6 | R average | DSR |
|---|---|---|---|---|---|---|---|---|
| 6 | 72.5 | 71.8 | 70.4 | 72.6 | 73.5 | 80.4 | 73.5 | 4.8 |
| 8 | 93.8 | 94.4 | 92.0 | 92.9 | 96.4 | 96.5 | 94.3 | 2.0 |
| 12 | 100.1 | 97.6 | 102.2 | 98.1 | 102.9 | 98.4 | 99.9 | 2.2 |

Sample under test: full dose of 60mg Gliclazide tablet

| Hours | T1 | T2 | T3 | T4 | T5 | T6 | T average | DSR |
|---|---|---|---|---|---|---|---|---|
| 2 | 15.4 | 14.7 | 16.5 | 19.1 | 17.7 | 17.6 | 16.8 | 9.6 |
| 4 | 33.1 | 32.2 | 36.1 | 39.1 | 37.5 | 37.5 | 35.9 | 7.6 |
| 6 | 51.6 | 50.4 | 56.3 | 59.7 | 55.9 | 56.0 | 55.0 | 6.2 |
| 8 | 70.6 | 70.3 | 75.7 | 78.1 | 75.3 | 75.3 | 74.2 | 4.2 |
| 12 | 99.8 | 97.9 | 94.4 | 97.0 | 98.2 | 98.5 | 97.6 | 1.9 |

$f_2 = 43,6$

The Similarity Factor or f2 is calculated using the following formula:

$$f_2 = 50 \cdot \log \{ [ 1 + ( 1 / n \cdot \sum ( R_t - T_t )^2 ]^{-0.5} \cdot 100 \}$$

wherein:

$n$ =     Number of sampling times
$R_t$ =     Dissolution value of the reference sample at time t
$T_t$ =     Dissolution value of the sample under test at time t

As it can be seen from the tables, the releases of the full dose and of the half dose are not comparable to each other since the Similarity Factor (f2) is lower than 50. However, the half dose and full dose taken individually show releases that are comparable to those of the reference products such as Diamicron ®, 30mg and 60mg respectively.
Comparison of dissolution profiles of 60mg Gliclazide leading product of reference (Diamicron®) vs. 60mg Gliclazide Valpharma per tablet:

Dissolution medium: phosphate buffer pH 6.8, 1000 ml
Apparatus: stationary basket, 50 rpm

Reference sample: 60mg Gliclazide per tablet leading product of reference Diamicron® manufactured by Laboratoires Servier, Batch No.: 889195

| Hours | R1 | R2 | R3 | R4 | R5 | R6 | R7 | R8 | R9 | R10 | R11 | R12 | R average | DSR |
|---|---|---|---|---|---|---|---|---|---|---|---|---|---|---|
| 2 | 13.2 | 13.9 | 14.1 | 13.7 | 14.5 | 14.9 | 18.8 | 12.2 | 16.9 | 15.0 | 16.1 | 14.1 | 14.8 | 12.0 |
| 4 | 32.4 | 34.0 | 34.1 | 34.1 | 33.6 | 33.8 | 41.5 | 30.9 | 41.3 | 36.1 | 38.1 | 33.1 | 35.3 | 9.6 |
| 6 | 53.1 | 56.1 | 55.6 | 55.2 | 54.1 | 54.4 | 63.4 | 50.6 | 64.0 | 58.8 | 61.0 | 52.5 | 56.6 | 7.6 |
| 8 | 74.3 | 77.3 | 75.7 | 75.3 | 72.8 | 72.6 | 83.7 | 72.5 | 85.1 | 79.9 | 80.5 | 72.2 | 76.8 | 5.9 |
| 12 | 96.3 | 95.1 | 97.6 | 95.9 | 93.2 | 90.4 | 99.3 | 95.7 | 99.5 | 97.9 | 96.8 | 96.5 | 96.2 | 2.6 |

Sample according to the invention: Gliclazide 60 mg per tablet, Batch No.: 0000040851

| Hours | T1 | T2 | T3 | T4 | T5 | T6 | T7 | T8 | T9 | T10 | T11 | T12 | T average | DSR |
|---|---|---|---|---|---|---|---|---|---|---|---|---|---|---|
| 2 | 15.4 | 14.7 | 16.5 | 19.1 | 17.7 | 17.6 | 18.5 | 14.6 | 17.5 | 15.3 | 17.9 | 16.9 | 16.8 | 8.9 |
| 4 | 33.1 | 32.2 | 36.1 | 39.1 | 37.5 | 37.5 | 39.3 | 33.0 | 38.2 | 32.8 | 37.4 | 35.5 | 36.0 | 7.2 |
| 6 | 51.6 | 50.4 | 56.3 | 59.7 | 55.9 | 56.0 | 58.8 | 52.7 | 59.1 | 49.5 | 55.9 | 54.0 | 55.0 | 6.2 |
| 8 | 70.6 | 70.3 | 75.7 | 78.1 | 75.3 | 75.3 | 79.5 | 74.5 | 78.1 | 65.3 | 74.5 | 75.1 | 74.4 | 5.3 |
| 12 | 99.8 | 97.9 | 94.4 | 97.0 | 98.2 | 98.5 | 100.4 | 104.5 | 97.0 | 98.2 | 100.0 | 98.3 | 98.7 | 2.5 |

$f_2 = 82,8$

The analysis performed at different pHs show that the releases of this formulation are pH-dependent, in fact, at pH 4.5 and in water, releases that are very different from the ones at pH 6.8 are obtained. (see table below)

| MEDIUM | pH 4.5/1000 ml | Water/1000 ml |
|---|---|---|
| APPARATUS | Paddle 50 rpm + Stationary Basket | Paddle 50 rpm + Stationary Basket |
| | | |
| TIME | average | average |
| 2h | 12.9 | 15.6 |
| 4h | 29.9 | 36.2 |
| 6h | 45.3 | 51.6 |
| 8h | 55.3 | 60.8 |
| 10h | 57.6 | 65.3 |
| 12h | 59.8 | 66.6 |
| 16h | 60.4 | 68.0 |
| 20h | 60.4 | 68.8 |
| 24h | 60.9 | 68.8 |

## Claims

1. A modified-release pharmaceutical composition containing Gliclazide; said composition consisting of:

   Gliclazide, as active ingredient mixed with
   a mixture of hydroxypropylmethyl cellulose (HPMC) said mixture comprising at least two HPMCs having medium or high viscosity and at least one HPMC having low viscosity;
   other diluents, all soluble in water; and
   optionally a glidant and/or a lubricant;

   where medium viscosity means a viscosity not lower than 2000cps, high viscosity means a viscosity higher than 50000cps and low viscosity means a viscosity lower than 100cps;
   said composition being devoid of $CaHPO_4$.

2. The composition according to claim 1, wherein the mixture of HPMCs consists of three HPMCs, wherein one HPMC has a viscosity higher than 50000 cps, one HPMC has a viscosity from 2000 to 50000cps, and one HPMC has a viscosity lower than 100cps.

3. The composition according to claim 2, wherein the HPMCs are chosen from the group consisting of HPMC 5cps, HPMC 4000cps, HPMC 15000cps and HPMC 100000cps.

4. The composition according to any one of the claims 1-3, wherein the mixture of HPMCs is present in percentages in the range from 20% to 40% w/w with respect to the total weight of the composition.

5. The composition according to any one of the claims 1-4, wherein the water-soluble diluents are chosen from the group consisting of polyalcohols and mixtures thereof, preferably mannitol, maltodextrin, sorbitol, isomalt and mixtures thereof.

6. The composition according to any one of the claims 1-5, comprising a glidant and/or a lubricant.

7. The composition according to claim 6, wherein the glidant is magnesium stearate, sodium stearyl fumarate, stearic acid or mixtures thereof, and the lubricant is anhydrous colloidal silica.

8. The composition according to any one of the claims 1-7, consisting of the following % w/w composition with respect to the total:

| | |
|---|---|
| Gliclazide | 15-20%; |
| mixture of HPMCs | 20-40%; |
| water-soluble diluents | 40-60%; |
| glidant | 0-1%; |
| lubricant | 0-1%. |

9. The composition according to any one of the claims 1-8, in tablet form.

10. The composition according to claim 9, wherein said tablet contains a dosage of 60mg Gliclazide, said tablet being divisible into two or more dosing fractions.

11. A method of producing a composition according to any one of the claims 1-10, wherein Gliclazide is added to the mixture of HPMCs in order to obtain a mixture to which water-soluble diluents are added and finally, optionally, the glidant and the lubricant are added.

12. The method according to claim 11, wherein the mixture of all the components is subjected to direct compression or to induction granulation.

**Patentansprüche**

1. Gliclazid enthaltende pharmazeutische Zusammensetzung mit modifizierter Freisetzung; wobei die Zusammensetzung aus:

Gliclazid als Wirkstoff, gemischt mit einer Mischung aus Hydroxypropylmethylzellulose (HPMC), wobei die Mischung mindestens zwei HPMCs mit mittlerer oder hoher Viskosität und mindestens eine HPMC mit niedriger Viskosität umfasst;
anderen Verdünnungsmitteln, die alle in Wasser löslich sind;
gegebenenfalls einem Fließregulierungsmittel und/oder einem Schmiermittel besteht;
wobei mittlere Viskosität eine Viskosität von nicht weniger als 2000 cps bedeutet, hohe Viskosität eine Viskosität von höher als 50000 cps bedeutet und niedrige Viskosität eine Viskosität von niedriger als 100 cps bedeutet, wobei die Zusammensetzung frei von CaHPO4 ist.

2. Zusammensetzung nach Anspruch 1, wobei die Mischung aus HPMCs aus drei HPMCs besteht, wobei eine HPMC eine Viskosität von höher als 50000 cps aufweist, eine HPMC eine Viskosität von 2000 bis 50000 cps aufweist, und eine HPMC eine Viskosität von niedriger als 100 cps aufweist.

3. Zusammensetzung nach Anspruch 2, wobei die HPMCs aus der Gruppe bestehend aus HPMC 5 cps, HPMC 4000 cps, HPMS 15000 cps und HPMC 100000 cps ausgewählt sind.

4. Zusammensetzung nach einem der Ansprüche 1-3, wobei die Mischung aus HPMCs in Prozentsätzen im Bereich von 20% bis 40% w/w bezogen auf das Gesamtgewicht der Zusammensetzung vorhanden ist.

5. Zusammensetzung nach einem der Ansprüche 1-4, wobei die wasserlöslichen Verdünnungsmittel aus der Gruppe bestehend aus Polyalkoholen und deren Mischungen, vorzugsweise Mannit, Maltodextrin, Sorbit, Isomalt und deren Mischungen ausgewählt sind.

6. Zusammensetzung nach einem der Ansprüche 1-5, die ferner ein Fließregulierungsmittel und/oder ein Schmiermittel umfasst.

7. Zusammensetzung nach Anspruch 6, wobei das Fließregulierungsmittel Magnesiumstearat, Natriumstearylfumarat, Stearinsäure oder deren Mischungen ist, und das Schmiermittel wasserfreies kolloidales Silica ist.

8. Zusammensetzung nach einem der Ansprüche 1-7, bestehend aus der folgenden % w/w-Zusammensetzung in Bezug auf die Gesamtmenge:

| | |
|---|---|
| Gliclazid | 15-20% |
| Mischung aus HPMCs | 20-40% |
| wasserlösliche Verdünnungsmittel | 40-60% |
| Fließregulierungsmittel | 0-1%; |
| Schmiermittel | 0-1 %. |

9. Zusammensetzung nach einem der Ansprüche 1-8 in Tablettenform.

10. Zusammensetzung nach Anspruch 9, wobei die Tablette eine Dosis von 60 mg Gliclazid enthält, wobei die Tablette in zwei oder mehr Dosierungsfraktionen teilbar ist.

11. Verfahren zur Herstellung einer Zusammensetzung nach einem der Ansprüche 1-10, wobei der Mischung aus HPMCs Gliclazid zugegeben wird, um eine Mischung zu erhalten, der wasserlösliche Verdünnungsmittel und schließlich gegebenenfalls das Fließregulierungsmittel und das Schmiermittel zugegeben werden.

12. Verfahren nach Anspruch 11, wobei die Mischung aller Komponenten einer direkten Kompression oder einer Induktionsgranulation unterzogen wird.

## Revendications

1. Composition pharmaceutique à libération modifiée contenant du gliclazide ; ladite composition étant constituée de :

gliclazide, en tant que principe actif mélangé avec
un mélange d'hydroxypropylméthylcelluloses (HPMC), ledit mélange comprenant au moins deux HPMC présentant une viscosité moyenne ou élevée et au moins une HPMC présentant une viscosité basse ;
autres diluants, tous solubles dans l'eau ; et
éventuellement un agent de glissement et/ou un lubrifiant ;
où viscosité moyenne signifie une viscosité qui n'est pas inférieure à 2000 cP, viscosité élevée signifie une viscosité supérieure à 50 000 cP et viscosité basse signifie une viscosité inférieure à 100 cP ;
ladite composition étant dépourvue de $CaHPO_4$.

2. Composition selon la revendication 1, dans laquelle le mélange des HPMC est constitué de trois HPMC, dans laquelle une HPMC présente une viscosité supérieure à 50 000 cP, une HPMC présente une viscosité de 2000 à 50 000 cP, et une HPMC présente une viscosité inférieure à 100 cP.

3. Composition selon la revendication 2, dans laquelle les HPMC sont choisies dans le groupe constitué de HPMC à 5 cP, HPMC à 4 000 cP, HPMC à 15 000 cP et HPMC à 100 000 cP.

4. Composition selon l'une quelconque des revendications 1 à 3, dans laquelle le mélange de HPMC est présent dans des pourcentages situés dans la plage de 20 % à 40 % p/p par rapport au poids total de la composition.

**5.** Composition selon l'une quelconque des revendications 1 à 4, dans laquelle les diluants solubles dans l'eau sont choisis dans le groupe constitué de polyols et de leurs mélanges, de préférence le mannitol, la maltodextrine, le sorbitol, l'isomalt et leurs mélanges.

**6.** Composition selon l'une quelconque des revendications 1 à 5, comprenant un agent de glissement et/ou un lubrifiant.

**7.** Composition selon la revendication 6, dans laquelle l'agent de glissement est le stéarate de magnésium, le stéarylfumarate de sodium, l'acide stéarique ou leurs mélanges, et le lubrifiant est la silice colloïdale anhydre.

**8.** Composition selon l'une quelconque des revendications 1 à 7, constituée des % p/p de la composition suivants par rapport au total :

| | |
|---|---|
| gliclazide | 15 à 20 % ; |
| mélange des HPMC | 20 à 40 % ; |
| diluants solubles dans l'eau | 40 à 60 % ; |
| agent de glissement | 0 à 1 % ; |
| lubrifiant | 0 à 1 %. |

**9.** Composition selon l'une quelconque des revendications 1 à 8, sous forme de comprimé.

**10.** Composition selon la revendication 9, dans laquelle ledit comprimé contient un dosage de 60 mg de gliclazide, ledit comprimé étant divisible en deux fractions de dosage ou plus.

**11.** Procédé de production d'une composition selon l'une quelconque des revendications 1 à 10, dans lequel le gliclazide est ajouté au mélange des HPMC afin d'obtenir un mélange auquel des diluants solubles dans l'eau sont ajoutés et finalement, éventuellement, l'agent de glissement et le lubrifiant sont ajoutés.

**12.** Procédé selon la revendication 11, dans lequel le mélange de tous les composants est soumis à une compression directe ou à l'induction d'une granulation.

Dissolution profile of 60 mg Glicazide according to the invention, half dose vs. full dose

FIG. 1

Dissolution profile of 60 mg Glicazide according to the invention vs. Leading Product of reference

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0018373 A1
- WO 2006123213 A
- WO 2006061697 A
- WO 2008062470 A

- WO 03026637 A
- US 2009238870 A
- EP 2103302 A

**Non-patent literature cited in the description**

- **LABANA et al.** *J. Chem. Pharm. Res.,* 2011, vol. 3 (3), 348-352